# EUROPÄISCHE PATENTSCHRIFT

(11) **EP 2 703 760 B1**
(45) Veröffentlichungstag und Bekanntmachung des Hinweises auf die Patenterteilung: **04.03.2015**
(21) Anmeldenummer: 13003676.7
(22) Anmeldetag: 23.07.2013
(51) Int. Cl.: F27B 17/02, A61C 13/20, F27D 1/18

(54) **Dentalofen zum Sintern eines Zahnersatzes**
Dental oven for sintering a dental prosthesis
Four dentaire pour le frittage d'une prothèse dentaire

(30) Priorität: 30.08.2012 AT 9462012
(43) Veröffentlichungstag der Anmeldung: 05.03.2014
(73) Patentinhaber: STEGER, Heinrich, 39031 Bruneck (IT)
(72) Erfinder: STEGER, Heinrich, 39031 Bruneck (IT)
(74) Vertreter: Torggler, Paul Norbert

(56) Entgegenhaltungen:
- WO-A1-2011/020688
- DE-A1- 4 308 244
- DE-C1- 4 105 845

## Beschreibung

Die Erfindung betrifft einen Dentalofen zum Sintern eines Zahnersatzes.

Auf dem Gebiet der Zahntechnik, im Speziellen der Herstellung von dentalen Werkstücken (Zahnersatz), sind schon seit vielen Jahren diverse Vorrichtungen bekannt. Eine Komponente die Zahntechniker dazu benötigen ist der Sinterofen wie z.B. DE 4105845 C1 zeigt. In diesem wird der vorher aus Keramik oder aus einem anderen Material gefertigte Zahnersatz eingebracht und durch Aufheizen gesintert. Nach dem Sintervorgang können weitere Vorgänge (z.B. Schleifen, usw.) durch den Zahntechniker durchgeführt werden.

Für ein verbessertes und rückstandsfreieres Sintern ist es zudem schon seit Jahren bekannt, den Zahnersatz in einem Vakuum zu sintern. Dazu gibt es bereits die verschiedensten Vakuumsinteröfen, die allerdings alle recht aufwändig zu handhaben sind und vor allem teuer in der Anschaffung sind. Zahnlabore benötigen deshalb zum einen herkömmliche Sinteröfen und zum anderen zusätzlich Vakuumsinteröfen, um sie entsprechend den Anforderungen einsetzen zu können.

Um nicht unbedingt einen kompletten, separaten Vakuumsinterofen anschaffen zu müssen, ist es aus der WO 2011/020688 A1 bekannt, einen herkömmlichen Sinterofen zu verwenden, wobei ein Quarzbehälter einen Sinterraum bildet der mit Schutzgas flutbar ist. Recht aufwändig bei dieser Art eines Sinterofens ist, dass der Quarzbehälter von unten in das Gehäuse eingeführt werden muss. Zudem ist nicht angegeben wie ein Sintern ohne Quarzbehälter erfolgt.

Die gleichen Nachteile gelten auch für die DE 43 08 244 A1, welche einen Hochtemperaturofen für die Wärmebehandlung und Sinterung unterschiedlicher Produkte zeigt. Es ist nicht angegeben, dass dieser Ofen auch ohne die Verwendung des napfförmigen Gefäßes verwendet werden könnte.

Die Aufgabe der vorliegenden Erfindung besteht daher darin, einen gegenüber dem Stand der Technik verbesserten Dentalofen zu schaffen. Insbesondere soll es für einen Anwender einfacher gemacht werden, die Vorteile des Vakuumsintems und des normalen Sinterns zu kombinieren. Zudem sollen ein einfaches Nachrüsten und ein schnelles Umrüsten von einem herkömmlichen Sinterofen auf einen Vakuumsinterofen möglich sein.

Dies wird durch ein Dentalofen-Set nach Anspruch 1 gelöst. Demnach ist vorgesehen, dass das Dentalofen-Set zum Sintern eines Zahnersatzes ein Gehäuse, das einen Heizraum zum Großteil umgibt, eine Verschlussvorrichtung für das Gehäuse zum Verschließen des Heizraumes und einen vom Gehäuse gesonderten, an diesem lösbar anbringbaren Aufsatz zum Verschließen des Heizraums, mit einem in den Heizraum ragenden Vakuumbehälter aufweist, wobei der Heizraum wahlweise mit der Verschlussvorrichtung oder mit dem Aufsatz verschließbar ist. Somit kann ein Zahntechniker eigenständig auf einen Vakuumsinterofen aufrüsten und bei Bedarf diese Applikation auch wieder entfernen, um wieder bei normalen Sinterungen ohne Schutzgas oder Vakuum die volle Brennraumgröße nutzen zu können.

Die Verschlussvorrichtung kann in Form eines Deckels, eines Stöpsels oder eines Aufsatzes ausgebildet sein. Für eine einfache Handhabung ist aber bevorzugt vorgesehen, dass die Verschlussvorrichtung in Form einer am Gehäuse bewegbar, insbesondere verschwenkbar, anbringbaren Türe ausgebildet ist.

Grundsätzlich ist es möglich, dass die Verschlussvorrichtung bzw. die Türe beim Vakuumsintem komplett vom Gehäuse abgenommen wird. Bevorzugt ist aber vorgesehen, dass bei durch den Aufsatz verschlossenem Heizraum die Türe (bzw. Verschlussvorrichtung) beim Sintern des im Vakuumbehälter angeordneten Zahnersatzes offen bleibt.

Durch die Ausbildung des Dentalofens in Form eines Sets sind zwei vorteilhafte Verschlussvarianten gegeben. Gemäß einem bevorzugten Ausführungsbeispiel ist demnach vorgesehen, dass das Dentalofen-Set zum Verschließen des Heizraums einen dem Gehäuse zugeordneten, gehäuseseitigen Kontaktbereich, einen der Türe zugeordneten, türseitigen (bzw. verschlussvorrichtungsseitigen) Kontaktbereich und einen dem Aufsatz zugeordneten, aufsatzseitigen Kontaktbereich aufweist, wobei bei am Gehäuse angebrachter Türe der Heizraum über den gehäuseseitigen Kontaktbereich und den türseitigen Kontaktbereich verschließbar ist und bei am Gehäuse angebrachtem Aufsatz der Heizraum über den gehäuseseitigen Kontaktbereich und den aufsatzseitigen Kontaktbereich verschließbar ist.

Um ein Entweichen der aufgeheizten Luft aus dem Heizraum beim Sintern soweit wie möglich zu unterbinden, ist bevorzugt vorgesehen, dass im Kontaktbereich zwischen Gehäuse und Türe bzw. zwischen Gehäuse und Aufsatz eine Dichtung (vorzugsweise in Form eines Dichtrings) vorgesehen ist. Grundsätzlich kann diese Dichtung bzw. dieser Dichtring immer am Gehäuse angeordnet bleiben. Bevorzugt ist jedoch vorgesehen, dass der türseitige Kontaktbereich einen Dichtring aufweist. In gleicher Art und Weise kann auch vorgesehen, dass der aufsatzseitige Kontaktbereich einen Dichtring aufweist.

Prinzipiell kann der Vakuumbehälter eine quaderförmige oder zylindrische Form aufweisen. Für einen konstruktiv einfachen Aufbau des Aufsatzes samt Vakuumbehälter ist aber bevorzugt vorgesehen, dass der Vakuumbehälter einen glockenförmigen, in den Heizraum ragenden Grundkörper und einen über Dichtelemente vakuumdicht auf den Grundkörper lösbar aufsetzbaren Deckel aufweist. Dabei kann weiters bevorzugt vorgesehen sein, dass der Vakuumbehälter über eine Leitung mit einer Vakuumerzeugungsvorrichtung verbunden ist. Der Vakuumbehälter kann aus Keramik (Aluminiumoxid, Siliziumkarbid, Zirkonoxid), Hartmetall, Wolfram, Wolframkarbid, Platin, usw. oder Mischungen daraus bestehen.

Besonders bevorzugt ist der Vakuumbehälter in Form eines Aluminiumoxidbehälters ausgebildet. Die Vorteile gegenüber einem Quarzrohr bestehen dabei darin, dass eine Temperaturbeständigkeit bis 1900° C gegeben ist, keine Beschichtung mit Bornitrid notwendig ist, keine inerten Kugeln notwendig sind und ein solcher Aluminiumoxidbehälter auch horizontal verwendet werden kann.

Im Gehäuse ist bevorzugt eine Heizvorrichtung angeordnet, die in den Heizraum ragende Heizstäbe aufweist. Für die Temperaturüberwachung kann am Gehäuse eine in den Heizraum mündende bzw. ragende Temperaturmessvorrichtung angeordnet sein.

Die Befestigung des Aufsatzes am Gehäuse kann über Magnete erfolgen. Die Türe ist bevorzugt über eine Verriegelungsvorrichtung in Schließstellung am Gehäuse verriegelbar.

Um das umständliche vertikale Beschicken des Dentalofens mit einem Vakuumbehälter zu vermeiden ist besonders bevorzugt vorgesehen, dass der Dentalofen bzw. dessen Gehäuse eine horizontale Aufstandsfläche aufweist, wobei der Aufsatz im Wesentlichen parallel zur Aufstandsfläche in den Heizraum des Dentalofens einschiebbar ist.

Weitere Einzelheiten und Vorteile der vorliegenden Erfindung werden anhand der Figurenbeschreibung unter Bezugnahme auf die in den Zeichnungen dargestellten Ausführungsbeispiele im Folgenden näher erläutert. Darin zeigen:
- Fig. 1: schematisch einen Sinterofen mit Gehäuse und Türe,
- Fig. 2: schematisch ein Dentalofen-Set mit Gehäuse, Türe und Aufsatz,
- Fig. 3: eine 3D-Ansicht eines offenen Sinterofens mit Gehäuse und Türe,
- Fig. 4: einen Schnitt durch einen geschlossenen Sinterofen,
- Fig. 5: eine 3D-Ansicht eines Dentalofen-Sets,
- Fig. 6: eine Frontansicht des Dentalofen-Sets,
- Fig. 7: eine Seitenansicht des Dentalofen-Sets und
- Fig. 8: einen Schnitt durch den Dentalofen mit abgebrachtem Aufsatz.

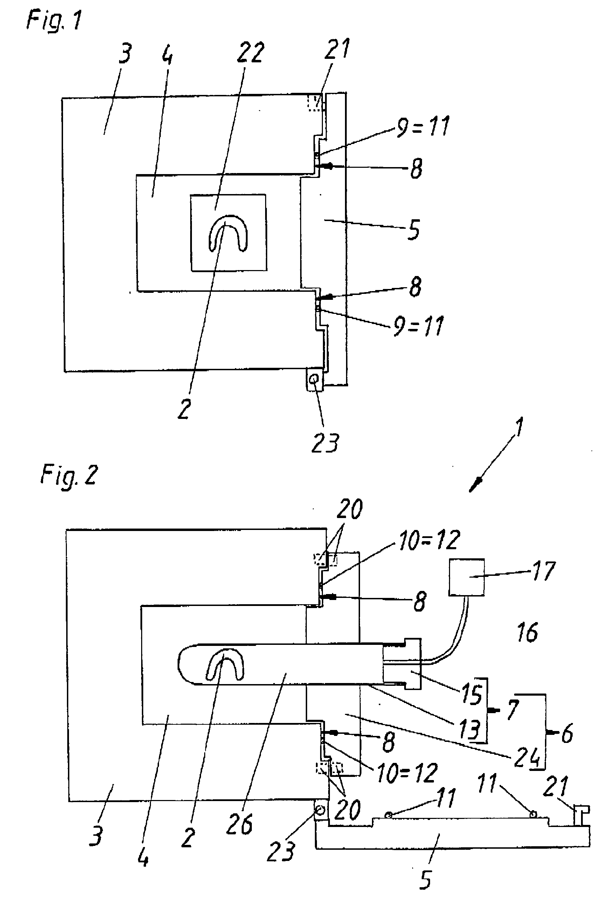

Fig. 1 zeigt schematisch einen horizontalen Schnitt durch einen geschlossenen Dentalofen beim Sintern eines Zahnersatzes 2 in aufgeheizter Luft im Heizraum 4. Der Zahnersatz 2 ist dabei auf einem Schamottblock 22 gelagert. Der Heizraum 4 ist vom Gehäuse 3 und von der Türe 5 (Verschlussvorrichtung) umgeben. Die Türe 5 ist über das Schwenkscharnier 23 um eine vertikale Achse bewegbar am Gehäuse 3 gelagert und über die Verriegelungsvorrichtung 21 am Gehäuse 3 in Schließstellung verriegelt. Der Verschluss bzw. die Abdichtung des Heizraums 4 erfolgt über den am Gehäuse 3 ausgebildeten gehäuseseitigen Kontaktbereich 8 und den an der Türe 5 ausgebildeten türseitigen Kontaktbereich 9. Dieser türseitige Kontaktbereich 9 ist in Form eines Dichtrings 11 ausgebildet. Prinzipiell kann auf eine solche Dichtung auch verzichtet werden.

Der große Vorteil der vorliegenden Erfindung liegt darin, dass der Heizraum 4 wahlweise mit der Türe 5 oder mit dem Aufsatz 6 verschließbar ist. Demnach ist in Fig. 2 gezeigt, dass die Türe 5 geöffnet ist und in einer an sich beliebigen, den Aufsatz 6 nicht störenden Offenstellung bleibt, während der Aufsatz 6 für das Vakuumsintern am Gehäuse 3 angebracht ist und den Heizraum 4 verschließt. Dieser Aufsatz 6 besteht zum einen aus der Aufsatzbasis 24 und zum anderen aus dem Vakuumbehälter 7. Dieser Vakuumbehälter 7 wiederum weist einen in den Heizraum 4 ragenden Grundkörper 13 und einen Deckel 15 auf. Im glockenförmigen Grundkörper 13 wird der gewünschte Zahnersatz 2 angeordnet, sodass einerseits die geheizte Luft im Heizraum 4 auf den Zahnersatz 2 wirkt und andererseits ein Schutz vor Oxidierung durch ein im Vakuumbehälter 7 erzeugtes Vakuum gegeben ist. Das Vakuum im Vakuumbehälter 7 wird von einer Vakuumerzeugungsvorrichtung 17 erzeugt, die über eine Leitung 16 mit dem im Vakuumbehälter 7 ausgebildeten Hohlraum 26 verbunden ist. Der Behälter 7 kann aber auch mit Schutzgas geflutet werden. Der Heizraum 4 selbst wird einerseits wieder über den gehäuseseitigen Kontaktbereich 8 und in diesem Fall andererseits mit dem aufsatzseitigen Kontaktbereich 10 verschlossen, vorzugsweise abgedichtet. Dieser aufsatzseitige Kontaktbereich 10 wird durch einen an der Aufsatzbasis 24 befestigten Dichtring 12 gebildet. Die Aufsatzbasis 24 wird über eine mechanische oder anderweitige Fixiervorrichtung, vorzugsweise über die Magnete 20, am Gehäuse 3 gehalten. Natürlich sind auch alternative Befestigungsvarianten möglich. Zwischen der Aufsatzbasis 24 und dem Grundkörper 13 des Vakuumbehälters 7 können auch Dichtelemente vorgesehen sein um ein Entweichen der geheizten Luft aus dem Heizraum 4 zu verhindern.

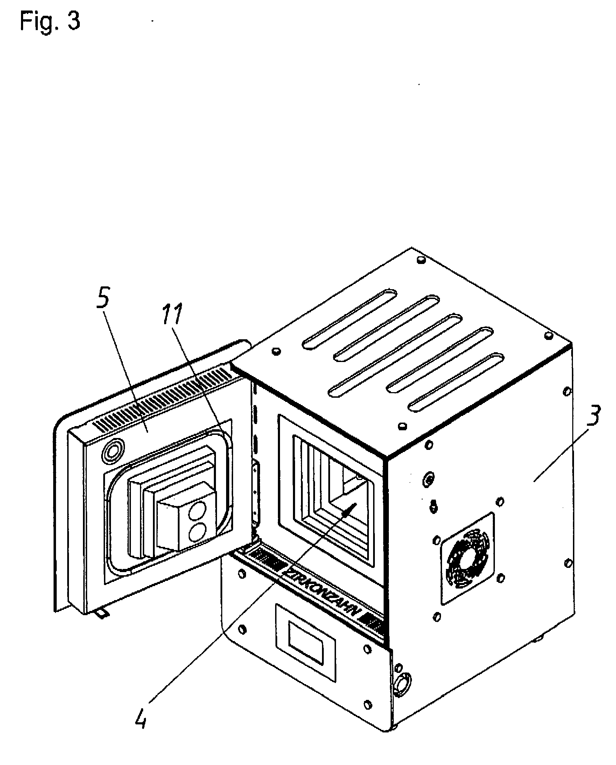

In Fig. 3 ist in einer 3D-Ansicht der Dentalofen mit Gehäuse 3 und Türe 5 ersichtlich, wobei der Blick in den Heizraum 4 frei ist. Die Abdichtung zwischen dem Gehäuse 3 und der Türe 5 erfolgt über den Dichtring 11.

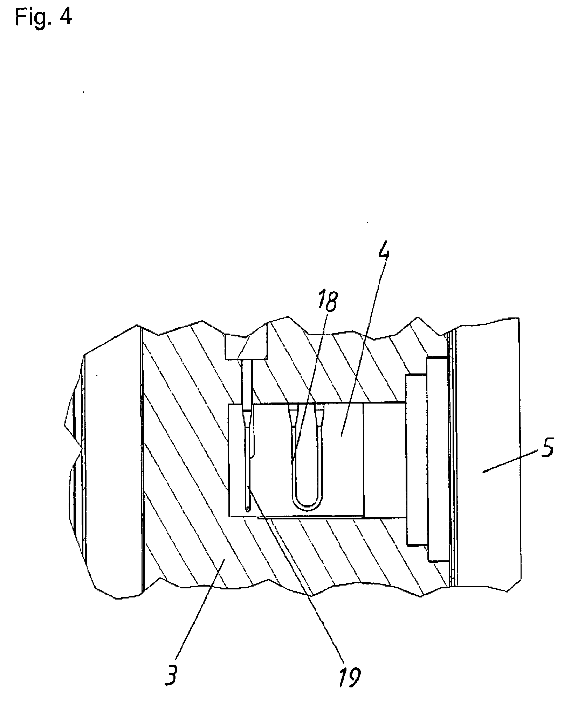

In Fig. 4 ist ein vertikaler Schnitt durch den Dentalofen und dessen Gehäuse 3 gezeigt, wobei ersichtlich ist, dass die Heizvorrichtung 18 (im Speziellen dessen Heizstäbe bzw. Heizelemente) und die Temperaturmessvorrichtung 19 in den von der Türe 5 verschlossenen Heizraum 4 ragen. Die Heizelemente müssen nicht wie im Bild dargestellt in den Heizraum 4 hineinragen sondern können auch horizontal, spiralförmig oder auf andere Art und Weise im Heizraum 4 angeordnet sein.

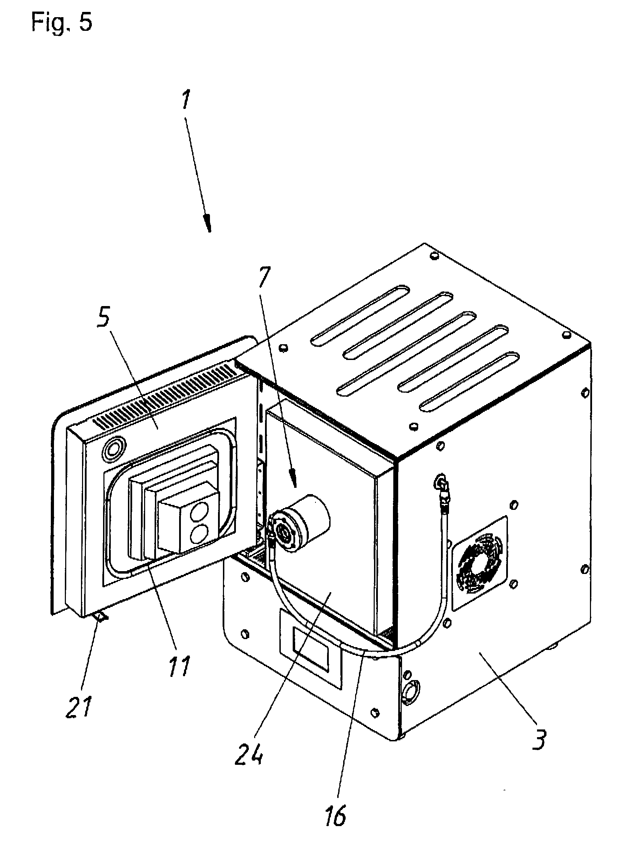

Fig. 5 zeigt das komplette Dentalofen-Set 1, bestehend aus den wesentlichen Komponenten Gehäuse 3, Aufsatz 6 und Türe 5. Bei geöffneter Türe 5 wird der Aufsatz 6 am Gehäuse 3 angebracht bzw. auf einfache Art und Weise in horizontaler Richtung eingeschoben, wodurch der Grundkörper 13 des Vakuumbehälters 7 in den Heizraum ragt. Der Hohlraum im Grundkörper 13 ist durch den Deckel 15 verschlossen, von welchem die Leitung 16 zu einer nicht näher dargestellten Vakuumerzeugungsvorrichtung 17 führt. Diese Vakuumerzeugungsvorrichtung 17 kann in das Gehäuse 3 integriert sein oder als separate Vorrichtung ausgebildet sein.

Fig, 6 zeigt eine zu Fig. 5 passende Frontansicht.

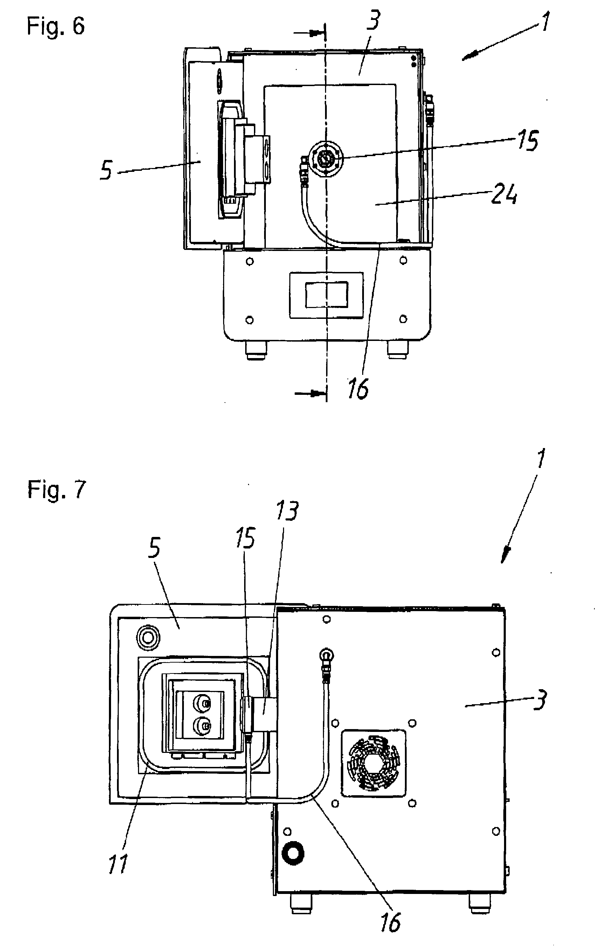

Fig. 7 zeigt das Dentalofen-Set 1 in einer Seitenansicht.

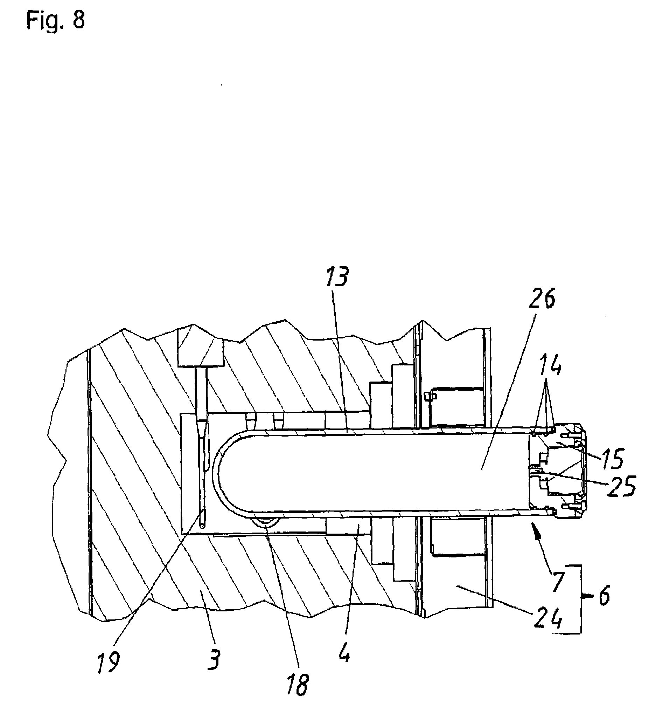

In Fig. 8 ist der in Fig. 6 eingezeichnete vertikale Schnitt durch das Dentalofen-Set 1 gezeigt. Dabei ist ersichtlich, dass der Grundkörper 13 weit in den Heizraum 4 hineinragt, wodurch die Doppelfunktion des Dentalofen-Sets 1 (normales Sintern bzw. wahlweise Vakuumsintem) besonders gut verdeutlicht ist. Der Grundkörper 13 ist über die Dichtelemente 14 vakuumdicht vom Deckel 15 verschlossen. Am Vakuumanschluss 25 wird die Leitung 16 angesetzt, sodass bei der Vakuumerzeugung die vorhandene Luft aus dem Hohlraum 26 abgesaugt wird. Die Temperaturmessvorrichtung 19 und die Heizvorrichtung 18 werden in gleicher Art und Weise wie beim Luftsintern eingesetzt.

Durch die vorliegende Erfindung ist somit ein Dentalofen-Set 1 geschaffen, welches wahlweise zum normalen Sintern und zum Vakuumsintern eingesetzt werden kann, wobei beim Vakuumsintern der Heizraum 4 anstelle der Türe 5 vom Aufsatz 6 samt Vakuumbehälter 7 verschlossen ist. Der größte Vorteil liegt dabei darin, dass eine schnelle Adaptierung und Umrüstung für einen Zahntechniker möglich ist, ohne einen kompletten, separaten Vakuumsinterofen anschaffen zu müssen.

## Patentansprüche

1. Dentalofen-Set (1) zum Sintern eines Zahnersatzes (2), mit
- einem Gehäuse (3), das einen Heizraum (4) zum Großteil umgibt,
- einer Verschlussvorrichtung für das Gehäuse (3) zum Verschließen des Heizraums (4) und
- einem vom Gehäuse (3) gesonderten, an diesem lösbar anbringbaren Aufsatz (6) zum Verschließen des Heizraums (4) mit einem in den Heizraum (4) ragenden Vakuumbehälter (7),
wobei der Heizraum (4) wahlweise mit der Verschlussvorrichtung oder mit dem Aufsatz (6) verschließbar ist.

2. Dentalofen-Set nach Anspruch 1, wobei die Verschlussvorrichtung in Form einer am Gehäuse (3) bewegbar, insbesondere verschwenkbar, anbringbaren Türe (5) ausgebildet ist.

3. Dentalofen-Set nach Anspruch 2, wobei bei durch den Aufsatz (6) verschlossenem Heizraum (4) die Türe (5) beim Sintern des im Vakuumbehälter (7) angeordneten Zahnersatzes (2) offen bleibt.

4. Dentalofen-Set nach Anspruch 2 oder 3, das zum Verschließen des Heizraums (4)
- einen dem Gehäuse (3) zugeordneten, gehäuseseitigen Kontaktbereich (8),
- einen der Türe (5) zugeordneten, türseitigen Kontaktbereich (9) und
- einen dem Aufsatz (6) zugeordneten, aufsatzseitigen Kontaktbereich (10) aufweist, wobei bei am Gehäuse (3) angebrachter Türe (5) der Heizraum (4) über den gehäuseseitigen Kontaktbereich (8) und den türseitigen Kontaktbereich (9) verschließbar ist und bei am Gehäuse (3) angebrachtem Aufsatz (6) der Heizraum (4) über den gehäuseseitigen Kontaktbereich (8) und den aufsatzseitigen Kontaktbereich (10) verschließbar ist.

5. Dentalofen-Set nach Anspruch 4, wobei der türseitige Kontaktbereich (9) einen Dichtring (11) aufweist.

6. Dentalofen-Set nach Anspruch 4 oder 5, wobei der aufsatzseitige Kontaktbereich (10) einen Dichtring (12) aufweist.

7. Dentalofen-Set nach einem der Ansprüche 1 bis 6, wobei der Vakuumbehälter (7) einen glockenförmigen, in den Heizraum (4) ragenden Grundkörper (13) und einen über Dichtelemente (14) vakuumdicht auf den Grundkörper (13) aufsetzbaren Deckel (15) aufweist.

8. Dentalofen-Set nach einem der Ansprüche 1 bis 7, wobei der Vakuumbehälter (7) über eine Leitung (16) mit einer Vakuumerzeugungsvorrichtung (17) verbunden ist.

9. Dentalofen-Set nach einem der Ansprüche 1 bis 8, mit einer in den Heizraum (4) ragenden Heizvorrichtung (18).

10. Dentalofen-Set nach einem der Ansprüche 1 bis 9, wobei am Gehäuse (3) eine in den Heizraum (4) ragende Temperaturmessvorrichtung (19) angeordnet ist.

11. Dentalofen-Set nach einem der Ansprüche 1 bis 10, wobei der Aufsatz (6) über Magnete (20) am Gehäuse (3) befestigbar ist.

12. Dentalofen-Set nach einem der Ansprüch 2 bis 11, wobei die Türe (5) über eine Verriegelungsvorrichtung (21) am Gehäuse (3) in Schließstellung verriegelbar ist.

## Claims

1. A dental oven set (1) for sintering a dental prosthesis (2) comprising
- a housing (3) which for the large part surrounds a heating chamber (4),
- a closure device for the housing (3) for closing the heating chamber (4), and
- an attachment (6) which is separate from the housing (3) and which can be releasably mounted thereto for closing the heating chamber (4) with a vacuum container (7) projecting into the heating chamber (4),
wherein the heating chamber (4) is selectively closable with the closure device or with the attachment (6).

2. A dental oven set according to claim 1 wherein the closure device is in the form of a door (5) which can be mounted to the housing (3) moveably, in particular pivotably.

3. A dental oven set according to claim 2 wherein with the heating chamber (4) closed by the attachment (6) the door (5) remains open upon sintering of the dental prosthesis (2) arranged in the vacuum container (7).

4. A dental oven set according to claim 2 or claim 3 which for closing the heating chamber (4) has
- a housing-side contact region (8) associated with the housing (3),
- a door-side contact region (9) associated with the door (5), and
- an attachment-side contact region (10) associated with the attachment (6),
wherein with the door (5) mounted to the housing (3) the heating chamber (4) is closable by way of the housing-side contact region (8) and the door-side contact region (9) and with the attachment (6) mounted to the housing (3) the heating chamber (4) is closable by way of the housing-side contact region (8) and the attachment-side contact region (10).

5. A dental oven set according to claim 4 wherein the door-side contact region (9) has a sealing ring (11).

6. A dental oven set according to claim 4 or claim 5 wherein the attachment-side contact region (10) has a sealing ring (12).

7. A dental oven set according to one of claims 1 to 6 wherein the vacuum container (7) has a bell-shaped main body (13) projecting into the heating chamber (4) and a cover (15) which can be fitted on to the main body (13) vacuum-tightly by way of sealing elements (14).

8. A dental oven set according to one of claims 1 to 7 wherein the vacuum container (7) is connected to a vacuum generating device (17) by way of a line (16).

9. A dental oven set according to one of claims 1 to 8 comprising a heating device (18) projecting into the heating chamber (4).

10. A dental oven set according to one of claims 1 to 9 wherein a temperature measuring device (19) which projects into the heating chamber (4) is arranged on the housing (3).

11. A dental oven set according to one of claims 1 to 10 wherein the attachment (6) can be fixed to the housing (3) by way of magnets (20).

12. A dental oven set according to one of claims 2 to 11 wherein the door (5) is lockable to the housing (3) in the closed position by way of a locking device (21).

## Revendications

1. Ensemble formant four dentaire (1) pour le frittage d'une prothèse dentaire (2), comprenant
- un carter (3) qui entoure en grande partie un espace de chauffage (4),
- un dispositif de fermeture pour le carter (3) pour fermer l'espace de chauffage (4) et
- une garniture (6) distincte du carter (3), pouvant être agencée de façon amovible sur celui-ci afin de fermer l'espace de chauffage (4) avec un conteneur sous vide (7) faisant saillie dans l'espace de chauffage (4),
dans lequel l'espace de chauffage (4) peut être fermé facultativement avec le dispositif de fermeture ou avec la garniture (6).

2. Ensemble formant four dentaire selon la revendication 1, dans lequel le dispositif de fermeture est conçu en forme de porte (5) pouvant être agencée de façon déplaçable, en particulier pivotante, sur le carter (3).

3. Ensemble formant four dentaire selon la revendication 2, dans lequel, l'espace de chauffage (4) étant fermé par la garniture (6), la porte (5) reste ouverte lors du frittage de la prothèse dentaire (2) disposée dans le conteneur sous vide (7).

4. Ensemble formant four dentaire selon la revendication 2 ou 3, qui présente pour fermer l'espace de chauffage (4)
- une zone de contact du côté du carter (8), affectée au carter (3),
- une zone de contact du côté de la porte (9), affectée à la porte (5), et
- une zone de contact du côté de la garniture (10), affectée à la garniture (6), dans lequel, la porte (5) étant agencée sur le carter (3), l'espace de chauffage (4) peut être fermé par la zone de contact du côté du carter (8) et la zone de contact du côté de la porte (9) et, avec la garniture (6) agencée sur le carter (3), l'espace de chauffage (4) peut être fermé par la zone de contact du côté du carter (8) et la zone de contact du côté de la garniture (10).

5. Ensemble formant four dentaire selon la revendication 4, dans lequel la zone de contact du côté de la porte (9) présente une bague d'étanchéité (11).

6. Ensemble formant four dentaire selon la revendication 4 ou 5, dans lequel la zone de contact du côté de la garniture (10) présente une bague d'étanchéité (12).

7. Ensemble formant four dentaire selon l'une des revendications 1 à 6, dans lequel le conteneur sous vide (7) présente un corps de base (13) en forme de cloche, faisant saillie dans l'espace de chauffage (4) et un couvercle (15) pouvant être posé sur le corps de base (13) en étant étanche au vide au moyen d'éléments d'étanchéité.

8. Ensemble formant four dentaire selon l'une des revendications 1 à 7, dans lequel le conteneur sous vide (7) est relié par une conduite (16) à un dispositif de production sous vide (17).

9. Ensemble formant four dentaire selon l'une des revendications 1 à 8, avec un dispositif de chauffage (18) faisant saillie dans l'espace de chauffage (4).

10. Ensemble formant four dentaire selon l'une des revendications 1 à 9, dans lequel un dispositif de mesure de température (19) faisant saillie dans l'espace de chauffage (4) est disposé sur le carter (3).

11. Ensemble formant four dentaire selon l'une des revendications 1 à 10, dans lequel la garniture (6) peut être fixée au carter (3) par des aimants (20).

12. Ensemble formant four dentaire selon l'une des revendications 2 à 11, dans lequel la porte (5) peut être verrouillée en position fermée au carter (3) par un dispositif de verrouillage (21).
